# EUROPEAN PATENT APPLICATION

(11) **EP 4 785 984 A1**
(43) Date of publication of application: **05.08.2026**
(21) Application number: 23959558.0
(22) Date of filing: 28.11.2023
(51) Int. Cl.: A61M 37/00, A61N 7/00, A61B 8/08

(54) **FOCUSED ULTRASOUND PROCESSING DEVICE AND DRUG DELIVERY METHOD USING SAME**

(30) Priority: 24.11.2023 KR 20230165313
(71) Applicant: IMGT CO., LTD, Gyeonggi-do 13605 (KR)
(72) Inventor: SON, Keon Ho, Seongnam-si Gyeonggi-do 13462 (KR); KIM, Dae Seung, Seoul 07524 (KR)
(74) Representative: Sander, Rolf
(86) International application number: PCT/KR2023/019290
(87) International publication number: WO 2025/110301

(57) **Abstract**

Disclosed are a focused ultrasound treatment device and a drug delivery method using the same. The drug delivery method according to an embodiment includes the steps of: determining treatment parameters for mechanical effects of focused ultrasound, transmitting focused ultrasound with the determined treatment parameters to tissue through a focused ultrasound transducer, generating cavitation in the tissue without by utilizing the mechanical effects of the transmitted focused ultrasound, temporarily creating micro-pores in the tissue through the generated cavitation, and delivering a pre-injected drug to the tissue through the created micro-pores.

## Description

### [Technical Field]

The present invention relates to diagnostic and therapeutic technologies using ultrasound, and more specifically, to image scanning and therapeutic technologies utilizing focused ultrasound (FUS).

### [Background Art]

Ultrasound signals may be used to treat biological tissues such as cancer, tumors, lesions, and the like. Treatment with ultrasound involves treating a lesion by outputting ultrasound signals to the lesion in the human body. Compared to conventional surgical procedures or chemical treatments such as chemotherapy, ultrasound treatment causes less trauma to the patient and enables non-invasive treatment. Examples of its application include liver cancer, bone sarcoma, breast cancer, pancreatic cancer, kidney cancer, soft tissue tumors, pelvic tumors, and the like.

Focused ultrasound (FUS) signals may also be used to treat tissues such as cancer, tumors, lesions, and the like. Treatment using focused ultrasound has primarily been developed mostly for thermal ablation techniques, which thermally ablate tissues by using thermal effects. However, thermal ablation may cause thermal damage and pain in surrounding tissues.

### [Disclosure]

### [Technical problem]

According to an embodiment, a focused ultrasound treatment device and a drug delivery method using the same are proposed, which enable the safe delivery of drug to biological tissues in a non-thermal and non-histotripsy manner without the use of microbubbles in drug delivery using focused ultrasound.

### [Technical Solution]

A drug delivery method according to an embodiment includes the steps of determining treatment parameters for mechanical effects of focused ultrasound, transmitting focused ultrasound with the determined treatment parameters to tissue through a focused ultrasound transducer, generating cavitation in the tissue through the transmitted focused ultrasound, temporarily creating micro-pores in the tissue through the generated cavitation, and delivering a pre-injected drug to the tissue through the created micro-pores.

In the step of determining the treatment parameters, the intensity of focused ultrasound may be determined to be 1.0 kW/cm² to 3.0 kW/cm².

In the step of determining the treatment parameters, the frequency of focused ultrasound may be determined to be 0.5 MHz to 3.0 MHz.

In the step of determining the treatment parameters, the duty cycle of focused ultrasound may be determined to be 10% or less.

The step of generating the cavitation may include generating stable cavitation in which bubbles created by the focused ultrasound oscillate in synchronization with the focused ultrasound, and inertial cavitation in which the bubbles collapse.

The drug delivery method may further include the steps of detecting a cavitation signal, caused by a cavitation phenomenon occurring in the tissue due to the transmitted focused ultrasound, using a cavitation sensor, and analyzing the detected cavitation signal to verify the mechanical effects of the focused ultrasound.

In the step of verifying the mechanical effects of the focused ultrasound, it may be checked whether a stable cavitation signal and an inertial cavitation signal are present in the detected cavitation signal, and it may be determined that the mechanical effects have occurred when both the stable and inertial cavitation signals are detected.

The drug delivery method may further include the steps of adjusting the treatment parameters of the focused ultrasound according to the results of cavitation signal analysis, and transmitting focused ultrasound with the adjusted treatment parameters to the tissue through the focused ultrasound transducer.

The drug delivery method may further include the step of steering the direction of a beam of focused ultrasound to be transmitted.

In the step of steering the direction of the beam, at least one of mechanical beam steering or electrical beam steering through a multi-channel array may be performed.

The drug delivery method may further include the step of controlling a position of an ultrasound treatment head through a positioning arm unit.

The drug delivery method may further include the steps of outputting an image ultrasound signal to the tissue through an image transducer, receiving an ultrasound echo signal reflected from the tissue, processing the received ultrasound echo signal to generate an ultrasound image signal, and analyzing the generated ultrasound image signal to verify the mechanical effects of the focused ultrasound.

According to another embodiment, a focused ultrasound treatment device includes a focused ultrasound transducer configured to transmit focused ultrasound to tissue, and a processor configured to determine treatment parameters for mechanical effects of the focused ultrasound, transmit the focused ultrasound with the determined treatment parameters to the tissue through a focused ultrasound transducer to generate cavitation in the tissue without microbubbles by utilizing the mechanical effects of the transmitted focused ultrasound, and create temporary micro-pores through the generated cavitation such that a pre-injected drug is delivered to the tissue through the created micro-pores.

### [Advantages Effects]

According to a focused ultrasound treatment device and a drug delivery method using the same in accordance with an embodiment, using the mechanical effects of focused ultrasound may enable the delivery of a drug to biological tissue in a safe, non-thermal, non-histotripsy manner, and without the use of microbubbles.

### [Description of Drawings]

FIG. 1 is a diagram illustrating the configuration of a focused ultrasound treatment device for drug delivery according to an embodiment of the present invention.
FIG. 2 is a flowchart illustrating a drug delivery method according to an embodiment of the present invention.
FIG. 3 is a diagram illustrating sonoporation using cavitation according to an embodiment of the present invention.
FIG. 4 illustrates a cavitation signal according to an embodiment of the present invention.
FIG. 5 illustrates an example of beam steering by a focused ultrasound transducer according to an embodiment of the present invention.

### [Mode of the Invention]

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. The same reference numerals refer to the same components throughout this disclosure.

In the following description of the embodiments of the present invention, if a detailed description of related known functions or configurations is determined to unnecessarily obscure the gist of the present invention, the detailed description thereof will be omitted herein. The terms described below are defined in consideration of the functions in the embodiments of the present invention, and these terms may be varied according to the intent or custom of a user or an operator. Therefore, the definitions of the terms used herein should follow contexts disclosed herein.

Hereinafter, embodiments of the present invention will be described in detail with reference to the accompanying drawings. However, the present invention may be realized in various forms, and the scope of the present invention is not limited to such embodiments. The embodiments of the present invention are provided to aid those skilled in the art in the explanation and the understanding of the present invention.

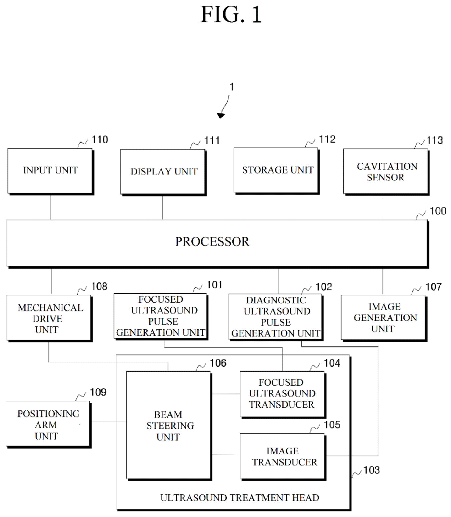

FIG. 1 is a diagram illustrating the configuration of a focused ultrasound treatment device for drug delivery according to an embodiment of the present invention.

One drug delivery method using focused ultrasound thermally induces vasodilation. However, the method of thermally inducing vasodilation is highly likely to cause thermal damage to surrounding tissues or major organs. Additionally, when tissues are thermally denatured, the penetration efficiency of therapeutic agents, such as anticancer drugs, decreases.

Another drug delivery method using focused ultrasound applies mechanical forces to microbubbles to induce cavitation, which facilitates drug delivery. However, this method has a limitation in that it requires the use of microbubbles. Moreover, commercially available microbubbles are inefficient due to their low stability and short lifespan.

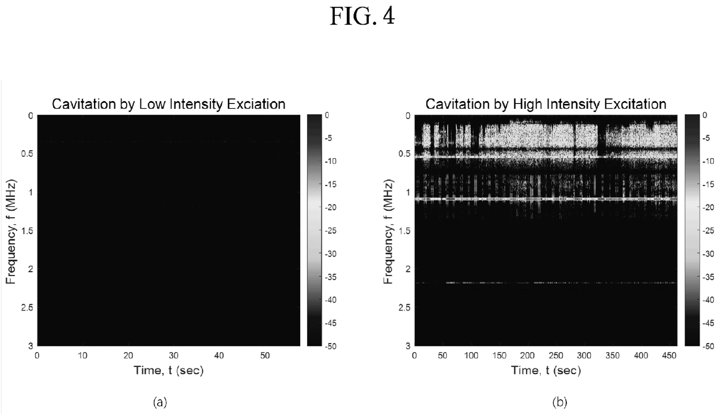

To address these issues, a focused ultrasound treatment device 1 according to an embodiment employs a safe, non-thermal, non-histotripsy method that does use require microbubbles. For example, the focused ultrasound treatment device 1 utilizes the mechanical effects of focused ultrasound to induce cavitation in human tissue without the use of microbubbles, thereby temporarily creating micro-pores in the tissue (sonoporation). Subsequently, the focused ultrasound treatment device 1 utilizes the acoustic streaming effect to deliver a drug through the micro-pores. The drug may include, for example, chemical therapeutic agents, gene therapeutic agents, biopharmaceuticals, genetic materials, and the like. Prior to transmitting focused ultrasound, the drug is injected through a blood vessel inside the body using a syringe or the like, and once the micro-pores are created, the drug present in the blood vessel is delivered to the tissue through the micro-pores. The focused ultrasound may be high-intensity focused ultrasound (HIFU). Even when focused ultrasound is applied to the body without microbubbles, the occurrence of cavitation may be verified using a cavitation sensor (see FIG. 4).

High-intensity focused ultrasound may cause gas molecules dissolved in a biofluid to aggregate to form gaseous bubbles. Alternatively, when the pressure of the fluid drops below the saturation vapor pressure due to high-intensity focused ultrasound, activated liquid molecules in the fluid may aggregate to form vaporous bubbles.

The phenomenon where the bubbles created in this manner oscillate in synchronization with the focused ultrasound is referred to as "stable cavitation." Additionally, the phenomenon in which these bubbles collapse is referred to as "inertial cavitation." Stable cavitation generates acoustic streaming around the bubbles, which exerts shear force on a vessel wall, creating a gap. Inertial cavitation generates micro-jets during bubble collapse, which in turn create micro-pores. The phenomenon in which micro-pores are created in cells and tissues due to these cavitation phenomena is called sonoporation. The cavitation phenomena refer to the formation of small bubbles within tissue caused by the action of negative and positive pressures resulting from pressure changes as focused ultrasound reaches the tissue. These bubbles repeatedly grow to their maximum size and collapse, leading to the destruction of cells within the tissue. Cavitation phenomena may generate impacts as the bubbles oscillate (stable cavitation) or collapse (inertial cavitation).

Hereafter, with reference to FIG. 1, the configuration of the focused ultrasound treatment device 1 possessing the aforementioned characteristics will be described.

Referring to FIG. 1, the focused ultrasound treatment device 1 includes a processor 100, a focused ultrasound pulse generation unit 101, a diagnostic ultrasound pulse generation unit 102, an ultrasound treatment head 103, an image generation unit 107, a mechanical drive unit 108, a positioning arm unit 109, an input unit 110, a display unit 111, a storage unit 112, and a cavitation sensor 113. The ultrasound treatment head 103 may include a focused ultrasound transducer 104, an image transducer 105, and a beam steering unit 106.

The focused ultrasound pulse generation unit 101 generates a pulse-shaped driving signal with a pulse repetition frequency (PRF) and a duty cycle, and transmits the driving signal to the focused ultrasound transducer 104.

The focused ultrasound transducer 104 converts an electrical waveform, which is the driving signal received from the focused ultrasound pulse generation unit 101 into focused ultrasound and outputs the converted focused ultrasound to tissue. At this time, the focused ultrasound may have an intensity of 1.0 kW/cm² to 3.0 kW/cm², a frequency of 0.5 MHz to 3.0 MHz, and a duty cycle of 10% or less. The duty cycle represents the proportion of the time that the signal is active during one period, expressed as a percentage.

The focused ultrasound transducer 104 may have a structure with a focused ultrasound radiation surface. The focused ultrasound transducer 104 generates focused ultrasound and focuses it on a treatment area. The focused ultrasound transducer 104 may have a multi-channel array structure composed of multiple components, and the multi-channel array structure may be in the form of an annular array with multiple therapeutic transducers arranged in an annular shape, or a random array with therapeutic transducers arranged in a random shape.

The image transducer 105 may output an image ultrasound signal based on a short pulse electrical signal, which is a driving signal received from the diagnostic ultrasound pulse generation unit 102, emits the image ultrasound signal into the tissue, and receives ultrasound echo signals reflected from the tissue. The image transducer 105 receives ultrasound echo signals until the next pulse is generated and transmits them to the processor 100.

The image transducer 105 may consist of a cylindrical-shaped casing containing a piezoelectric element and the like. The image transducer 105 may be configured as a phased array imaging transducer.

The ultrasound treatment head 103 may have a structure in which the image transducer 105 is positioned at the center and the focused ultrasound transducer 104 is arranged on the periphery. However, the arrangement of the image transducer 105 and the focused ultrasound transducer 104 is not limited to this configuration and may be modified in various ways.

The focused ultrasound treatment device 1 according to an embodiment may perform drug delivery using focused ultrasound generated by the focused ultrasound transducer 104 while simultaneously obtaining a diagnostic image using the image ultrasound from the image transducer 105.

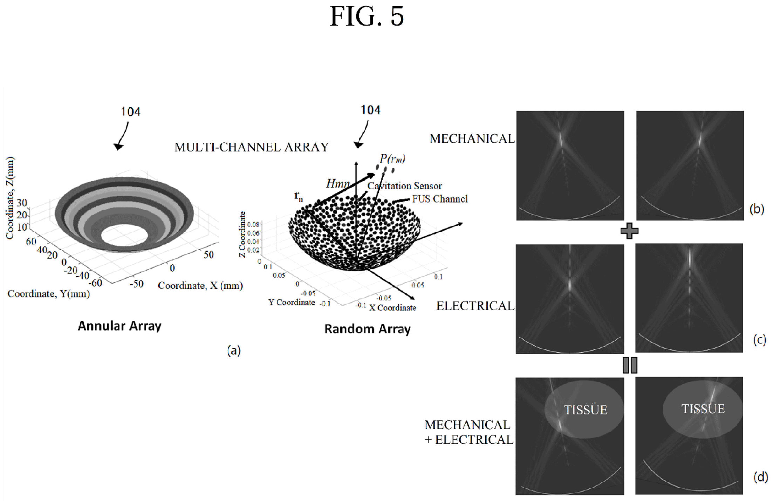

The beam steering unit 106 focuses a transmit or receive beam of ultrasound through mechanical and/or electronic beam steering. At this time, the beam steering unit 106 may steer the beam direction to vary the ultrasound focal region. The beam steering unit 106 may also perform hybrid beam steering, which combines mechanical steering with electronic steering. The beam steering unit 106 may use the multi-channel array structure of the focused ultrasound transducer 104 for mechanical and/or electronic beam steering. Examples of beam steering by the beam steering unit 106 will be described below with reference to FIG. 5.

The image generation unit 107 processes the ultrasound echo signals received from the image transducer 105 and generates an ultrasound image based on the processed signals. The generated ultrasound image may be displayed on a screen via the display unit 111. For signal processing, the image generation unit 107 may include a low noise amplifier (LNA) and an analog-to-digital converter (ADC).

The mechanical drive unit 108 drives the beam steering unit 106 to enable mechanical beam steering of the focused ultrasound transducer 104 and the image transducer 105.

The positioning arm unit 109 may move the ultrasound treatment head 103, which includes the focused ultrasound transducer 104 and the image transducer 105, to ensure that the ultrasound treatment head 103 is positioned accurately and stably on a target. To this end, the positioning arm unit 109 may include mechanisms for tilting and rotation. The positioning arm unit 109 may individually tilt or rotate the focused ultrasound transducer 104 or the image transducer 105 and may tilt or rotate the ultrasound treatment head 103 that includes the focused ultrasound transducer 104 and the image transducer 105. To this end, the positioning arm unit 109 may have three or more degrees of freedom.

The input unit 110 receives a user operation signal. To this end, the input unit 110 may include a user interface. The display unit 111 outputs the ultrasound image. The storage unit 112 stores received signals and information necessary for the operation of the processor 100. The storage unit 112 may store information required for signal analysis by the processor 100 or the information analyzed by the processor 100.

The processor 100 determines treatment parameters for the mechanical effects of focused ultrasound and controls the focused ultrasound transducer 104 to transmit focused ultrasound with the determined parameters to tissue. At this time, cavitation is generated in the tissue without microbubbles by utilizing the mechanical effects of the transmitted focused ultrasound, and micro-pores are temporarily created through the generated cavitation. Subsequently, a pre-injected drug is delivered to the tissue through the generated micro-pores.

The cavitation sensor 113 detects a cavitation signal from the tissue caused by a cavitation phenomenon induced by the focused ultrasound. The cavitation signal is used to verify the mechanical effects of the focused ultrasound necessary to induce cavitation in the tissue without the use of microbubbles. The processor 100 may analyze the cavitation signal detected by the cavitation sensor 113 to verify the mechanical effects of the focused ultrasound. At this time, the processor 100 may check whether a stable cavitation signal and an inertial cavitation signal are present in the detected cavitation signal. If both types of cavitation signals are detected, the processor may determine that mechanical effects have occurred. An example of cavitation signal analysis will be described below with reference to FIG. 4.

According to the results of the cavitation signal analysis, the processor 100 may adjust the parameters of the focused ultrasound. For example, the processor 100 may compare a detected cavitation value with a predetermined cavitation limit value. If the detected cavitation value is greater than or equal to the predetermined cavitation limit value, the processor may adjust the parameters of the focused ultrasound to reduce the detected cavitation value. Also, the processor 100 may compare the cavitation detected value with a cavitation threshold value. If the detected cavitation value is less than or equal to a predetermined cavitation threshold value, the processor may adjust the parameters of the focused ultrasound to increase the detected cavitation value. The cavitation limit value is higher than the cavitation threshold value. Setting values for the cavitation limit value and the cavitation threshold value may be determined according to the type of biological tissue and the characteristics of the focused ultrasound, and may also be set by receiving a user operation signal.

The processor 100 may determine the parameters of the focused ultrasound to prevent the occurrence of a shock scattering effect within biological tissue. The shock scattering effect occurs due to the interaction between bubbles and focused ultrasound, causing shocks to occur at points outside the focal position, potentially damaging unintended areas of biological tissue. Accordingly, the processor 100 may determine the parameters of the focused ultrasound to prevent the occurrence of such shock scattering effects.

The parameters of the focused ultrasound may include acoustic pressure, waveform, output duration, frequency, duty cycle, and the like. By adjusting the parameters of the focused ultrasound, the intensity and pressure of the focused ultrasound may be controlled.

The processor 100 may determine focused ultrasound parameters to maximize the mechanical effects of the focused ultrasound. For example, the processor 100 controls the focused ultrasound transducer 104 to output focused ultrasound with a predetermined first frequency, and compares the detected cavitation value with the predetermined cavitation limit value. Subsequently, the processor may adjust the frequency of the focused ultrasound from the first frequency to a second frequency when the detected cavitation value is greater than or equal to the cavitation limit value. Then, the processor 100 may control the focused ultrasound transducer 104 to output focused ultrasound with the adjusted second frequency.

The processor 100 may verify the mechanical effects of the focused ultrasound by analyzing the ultrasound image generated by the image generation unit 107. For example, the processor 100 analyzes image signals during a first cycle to check the brightness value of the focal area within the first frequency range of the first cycle. At this time, it determines whether the brightness value of the focal area falls within a predetermined cavitation brightness range. If the brightness value is within the predetermined brightness range, the processor 100 may determine that the mechanical effects have occurred. Furthermore, the processor 100 may adjust the parameters of the focused ultrasound according to the results of the ultrasound image signal analysis.

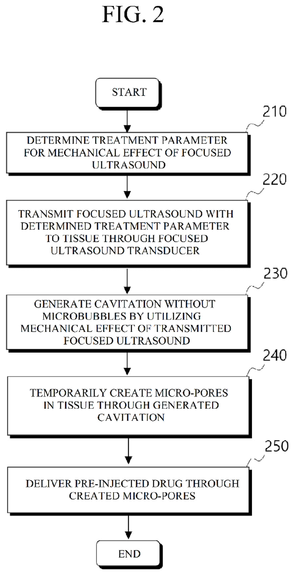

FIG. 2 is a flowchart illustrating a drug delivery method according to an embodiment of the present invention.

Referring to FIGS. 1 and 2, a drug is first injected into the body through blood vessels or the like. A syringe or similar device may be used for drug injection.

The focused ultrasound treatment device 1 determines the treatment parameters for the mechanical effects of focused ultrasound (210). The focused ultrasound may have an intensity of 1.0 kW/cm² to 3.0 kW/cm², a frequency of 0.5 MHz to 3.0 MHz, and a duty cycle of 10% or less.

Next, the focused ultrasound treatment device 1 transmits focused ultrasound with the determined treatment parameters to the tissue through the focused ultrasound transducer (220).

Subsequently, the focused ultrasound treatment device 1 generates cavitation in the tissue without the use of microbubbles by utilizing the mechanical effects of the transmitted focused ultrasound (230).

The focused ultrasound treatment device 1 then temporarily creates micro-pores in the tissue through the generated cavitation (240).

Subsequently, the focused ultrasound treatment device 1 facilitates the delivery of the pre-injected drug to the tissue through the created micro-pores (250).

Furthermore, the focused ultrasound treatment device 1 detects a cavitation signal, caused by the cavitation phenomenon occurring in the tissue due to the transmitted focused ultrasound, using the cavitation sensor 113, and analyzes the detected cavitation signal to verify the mechanical effects of the focused ultrasound. For example, the focused ultrasound treatment device 1 checks whether a stable cavitation signal and an inertial cavitation signal are present in the detected cavitation signal, and, when both types of cavitation signals are detected, it may determine that the mechanical effects have occurred.

A focused ultrasound treatment device 1 may adjust the treatment parameters of the focused ultrasound according to the results of the cavitation signal analysis and transmit the focused ultrasound with the adjusted treatment parameters to the tissue through the focused ultrasound transducer.

The focused ultrasound treatment device 1 may steer the direction of a beam of focused ultrasound to be transmitted. In this case, the focused ultrasound treatment device 1 may perform at least one of mechanical beam steering or electrical beam steering through a multi-channel array.

The focused ultrasound treatment device 1 may control the position of the ultrasound treatment head 103 using the positioning arm unit with three or more degrees of freedom.

The focused ultrasound treatment device 1 may verify the mechanical effects of the focused ultrasound by analyzing the ultrasound image generated by the image generation unit 107.

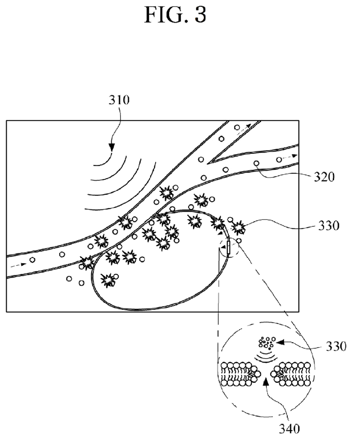

FIG. 3 is a diagram illustrating sonoporation using cavitation according to an embodiment of the present invention.

Referring to FIGS. 1 and 3, sonoporation occurs, creating micro-pores in cells and tissues through the cavitation phenomenon. For example, as shown in FIG. 3, when focused ultrasound 310 is transmitted via the focused ultrasound transducer, cavitation occurs in which bubbles 330 are formed in the tissue without microbubbles due to the mechanical effects of the transmitted focused ultrasound 310. Subsequently, micro-pores 340 are temporarily created in the tissue through the cavitation. Then, pre-injected drugs 320 in the body flow through blood vessels and are delivered to the tissue through the micro-pores 340.

FIG. 4 illustrates a cavitation signal according to an embodiment of the present invention.

More specifically, (a) shows a cavitation signal obtained at a frequency of 1.1 MHz under low-intensity excitation, while (b) shows a cavitation signal obtained at a frequency of 1.1 MHz under high-intensity excitation.

Referring to FIGS. 1 and 4, the mechanical effects of focused ultrasound may be verified through the cavitation signals.

Stable cavitation is associated with harmonic signals including harmonics (nf₀), sub-harmonics (1/2f₀), and ultra-harmonics ((2n+1)/2f₀), whereas inertial cavitation appears as broadband noise. Here, f₀ denotes a fundamental frequency.

Inertial cavitation occurs along with stable cavitation signals because stable cavitation progresses into inertial cavitation. Since the growth rates of individual bubbles in a bubble cluster vary, stable cavitation always occurs together with inertial cavitation.

FIG. 5 illustrates an example of beam steering by a focused ultrasound transducer according to an embodiment of the present invention.

More specifically, (a) illustrates a focused ultrasound transducer with a multi-channel array structure, (b) illustrates mechanical beam steering by the focused ultrasound transducer, (c) illustrates electrical beam steering by the focused ultrasound transducer, and (d) shows hybrid beam steering that combines the mechanical beam steering with electrical beam steering by the focused ultrasound transducer.

Referring to FIGS. 1 and 5, the focused ultrasound transducer 104 may have a multi-channel array structure composed of multiple components, and the multi-channel array structure may be in the form of an annular array with multiple therapeutic transducers arranged in an annular shape, or a random array with therapeutic transducers arranged in a random shape.

The focused ultrasound transducer 104 may perform at least one of mechanical beam steering or electrical beam steering through the multi-channel array. For example, as shown in (b), the focused ultrasound transducer 104 may perform mechanical beam steering. In another example, as shown in (c), the focused ultrasound transducer 104 may perform electrical beam steering. In another example, as shown in (d), the focused ultrasound transducer 104 may perform hybrid beam steering that combines mechanical beam steering with electrical beam steering.

Heretofore, the present invention has been described by focusing on the exemplary embodiments. It can be understood by those skilled in the art to which the present invention pertains that the present invention can be implemented in modified forms without departing from the essential feature of the present invention. Therefore, the disclosed embodiments should be considered as illustrative rather than determinative. The scope of the present invention is defined by the appended claims rather than by the foregoing description, and all differences within the scope of equivalents thereof should be construed as being included in the present invention.

## Claims

1. A drug delivery method using a focused ultrasound treatment device, comprising the steps of:
determining treatment parameters for mechanical effects of focused ultrasound;
transmitting focused ultrasound with the determined treatment parameters to tissue through a focused ultrasound transducer;
generating cavitation in the tissue without microbubbles by utilizing the mechanical effects of the transmitted focused ultrasound;
temporarily creating micro-pores in the tissue through the generated cavitation; and
delivering a pre-injected drug to the tissue through the created micro-pores.

2. The drug delivery method of claim 1, wherein the step of determining the treatment parameters comprises determining an intensity of focused ultrasound to be 1.0 kw/cm² to 3.0 kW/cm².

3. The drug delivery method of claim 1, wherein the step of determining the treatment parameters comprises determining a frequency of focused ultrasound to be 0.5 MHz to 3.0 MHz.

4. The drug delivery method of claim 1, wherein the step of determining the treatment parameters comprises determining a duty cycle of focused ultrasound to be 10% or less.

5. The drug delivery method of claim 1, the step of generating the cavitation comprises generating stable cavitation in which bubbles created by the focused ultrasound oscillate in synchronization with the focused ultrasound, and inertial cavitation in which the bubbles collapse.

6. The drug delivery method of claim 1, further comprising the steps of:
detecting a cavitation signal, caused by a cavitation phenomenon occurring in the tissue due to the transmitted focused ultrasound, using a cavitation sensor; and
analyzing the detected cavitation signal to verify the mechanical effects of the focused ultrasound.

7. The drug delivery method of claim 6, wherein the step of verifying the mechanical effects of the focused ultrasound comprises checking whether a stable cavitation signal and an inertial cavitation signal are present in the detected cavitation signal, and determining that the mechanical effects have occurred when both the stable and inertial cavitation signals are detected.

8. The drug delivery method of claim 6, further comprising the steps of:
adjusting the treatment parameters of the focused ultrasound according to results of cavitation signal analysis; and
transmitting focused ultrasound with the adjusted treatment parameters to the tissue through the focused ultrasound transducer.

9. The drug delivery method of claim 1, further comprising the step of steering a direction of a beam of focused ultrasound to be transmitted.

10. The drug delivery method of claim 9, wherein the step of steering the direction of a beam comprises performing at least one of mechanical beam steering or electrical beam steering through a multi-channel array.

11. The drug delivery method of claim 1, further comprising the step of controlling a position of an ultrasound treatment head through a portioning arm unit.

12. The drug delivery method of claim 1, further comprising the steps of:
outputting an image ultrasound signal to the tissue through an image transducer and receiving an ultrasound echo signal reflected from the tissue;
processing the received ultrasound echo signal to generate an ultrasound image signal; and
analyzing the generated ultrasound image signal to verify the mechanical effects of the focused ultrasound.

13. A focused ultrasound treatment device comprising:
a focused ultrasound transducer configured to transmit focused ultrasound to tissue; and
a processor configured to determine treatment parameters for mechanical effects of the focused ultrasound, transmit the focused ultrasound with the determined treatment parameters to the tissue through a focused ultrasound transducer to generate cavitation in the tissue without microbubbles by utilizing the mechanical effects of the transmitted focused ultrasound, and create temporary micro-pores through the generated cavitation such that a pre-injected drug is delivered to the tissue through the created micro-pores.
